# EUROPÄISCHE PATENTSCHRIFT

(11) **EP 0 464 617 B1**
(45) Veröffentlichungstag und Bekanntmachung des Hinweises auf die Patenterteilung: **06.09.1995**
(21) Anmeldenummer: 91110526.0
(22) Anmeldetag: 26.06.1991
(51) Int. Cl.: G01N 33/32

(54) **Farbübertragungsrolle**
Roller for transferring ink
Rouleau de transfert d'encre

(30) Priorität: 04.07.1990 DE 4021183
(43) Veröffentlichungstag der Anmeldung: 08.01.1992
(73) Patentinhaber: KOENIG & BAUER-ALBERT AKTIENGESELLSCHAFT, 97080 Würzburg (DE)
(72) Erfinder: Schneider, Georg, W-8700 Würzburg (DE)

(56) Entgegenhaltungen:
- DE-A- 3 520 831
- DE-B- 1 573 453
- DE-B- 2 917 519
- US-A- 3 063 285
- US-A- 3 881 349
- IGT MEDEDELINGENBLAD Nr. 2, September 1968, AMSTERDAM NL 'De IGT- Proefdruktoestellen A1, A2 en AC2 '

## Beschreibung

Die Erfindung betrifft ein Verfahren und eine Vorrichtung zur Beurteilung von auf Papier gedruckter Druckfarbe gemäß dem jeweiligen Oberbegriff der Patentansprüche 1 und 2.

Eine derartiges Verfahren und eine Vorrichtung zur Durchführung des Verfahrens gehen aus der DE-AS-29 17 519 hervor.
Diese Vorrichtung dient zum Abnehmen und Beurteilen eines Abschmierverhaltens von auf Papier gedruckter Druckfarbe. Es wird zum Abnehmen von Druckfarbe ein Abschmierer in Form einer Rolle, die einen farbfreundlichen Mantel aufweist, verwendet.

Zur Beurteilung des Abschmierverhaltens von frisch auf Papier gedruckter Farbe ist es bekannt, siehe z. B. Institutsbericht 1/1979 der Technischen Hochschule Darmstadt, Beitrag zur Messung des Trockengrades eines gedruckten Farbfilms an der laufenden Papierbahn, diese mittels einer Abschmierleiste zu verschmieren und die daraus resultierende Schmierspur zu untersuchen, wobei eine Abnahme der Leuchtdichte der Schmierspur im Verhältnis zur Leuchtdichte einer unbedruckten Fläche als Beurteilungskriterium herangezogen wird.
Zur Erzeugung einer aussagekräftigen Schmierspur ist eine aufwendige Vorrichtung notwendig. Die Verschmierung kann nur auf dem Produkt erfolgen, auf dem auch der Probedruck erfolgte. Außerdem muß die Schmierspur über die gesamte Schmierlänge untersucht werden.

Der Erfindung liegt die Aufgabe zugrunde, ein Verfahren und eine Vorrichtung zur Durchführung des Verfahrens zu schaffen, mittels dem in einfacher Weise das Abschmierverhalten von auf Papier gedruckter Farbe beurteilt werden kann.

Die Aufgabe wird durch die jeweiligen Merkmale der kennzeichnenden Teile des Anspruches 1 verfahrensgemäß und des Anspruches 2 vorrichtungsgemäß gelöst.

Das erfindungsgemäße Verfahren ist jederzeit und überall leicht anwendbar. Die zur Durchführung des Verfahrens angegebene Vorrichtung ist tragbar, kostengünstig herstellbar und leicht bedienbar, so daß jeder Drucker diese bei sich führen kann.

Bei der Anwendung des erfindungsgemäßen Verfahrens kann eine ausgesuchte Farbfläche oder Farblinie mit definierter Kraft übertragen werden, so daß nur die übertragene Farbfläche bzw. Farblinie untersucht werden muß.

Ein Ausführungsbeispiel der Erfindung ist in den Zeichnungen dargestellt und wird im folgenden beschrieben. Es zeigen:

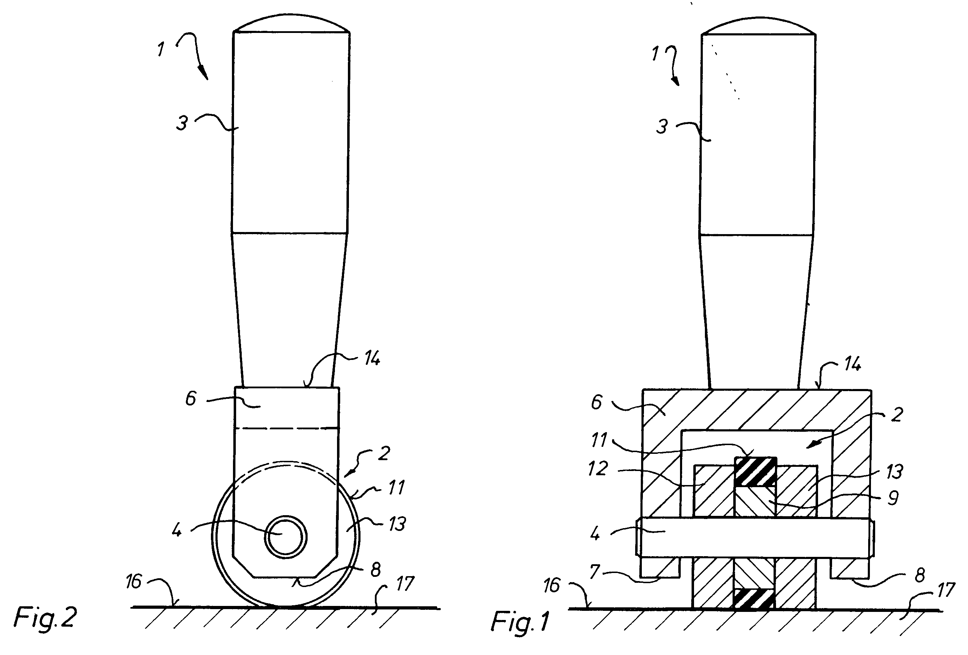

Fig. 1 eine Vorderansicht der erfindungsgemäßen Vorrichtung,
Fig. 2 eine Seitenansicht.

Das erfindungsgemäße Farbübertragungsmittel 1 besteht im Ausführungsbeispiel aus einer drehbar gelagerten Rolle 2, die zwecks einfacher Handhabung mit einem Handgriff 3 versehen ist. Die Rolle 2 sitzt drehfest auf einer Welle 4. Diese ist in einem nach unten offenen U-Profil-Träger 6 drehbar gelagert. Hierbei steht die Rolle 2 über die Enden 7, 8 des U-Profil-Trägers 6 hinaus. Die Rolle 2 besteht aus einem scheibenförmigen Mittelteil 9, welches mit einem farbfreundlichen, leicht deformierbaren Mantel 11 umgeben ist. Jeweils seitlich des scheibenförmigen Mittelteils 9 sind zwei weitere schwer deformierbare Scheiben 12, 13 angeordnet. Die Scheiben 12 und 13 können gegenüber dem Mantel 11 als starr und farbabstoßend bezeichnet werden. Ein Radius r (z. B. r = 17 mm) der Scheiben 12, 13 ist kleiner als ein Radius R (z. B. R = 18 mm) des Mantels 11. Der Handgriff 3 ist an einer Oberseite 14 des U-Profil-Trägers 6 befestigt.

Zur Durchführung des Verfahrens wird ein frisches Druckprodukt 16 auf eine feste Unterlage 17 (z. B. Meßtisch) gelegt. Auf dem Druckprodukt 16 wird ein relevanter Druckbereich oder eine relevante Drucklinie ausgesucht, vorzugsweise eine Volltonlinie. Nach einer festgesetzten Zeit T (z. B. T = 20 Sekunden) nach dem Druck wird die ausgesuchte Drucklinie mittels der Rolle 2 überrollt. Hierbei wird diese derart an das Druckprodukt 16 angedrückt, daß der Mantel 11 soweit komprimiert wird, bis dieser mit den Scheiben 12, 13 eine Auflagefläche bildet. Der Mantel 11 wird hierbei unter definiertem Druck mit der zu untersuchenden Druckstelle in Berührung gebracht. Die Druckfarbe der zu untersuchenden Stelle wird je nach Trocknungszustand mehr oder weniger von der Rolle 2 abgenommen und durch Fortführung der Rollbewegung an eine unbedruckte Stelle des Druckproduktes 16 oder eines Prüfbogens übertragen und abgelegt. Der so entstandene Abdruck wird nun visuell oder mittels eines Densitometers untersucht und ausgewertet. Die Farbdichte des Abdrucks gibt dem Drucker Aufschluß über die Abschmierneigung der verwendeten Druckfarbe in Zusammenhang mit dem verwendeten Druckpapier.

### Teileliste

- 1: Farbübertragungsmittel
- 2: Rolle
- 3: Handgriff
- 4: Welle
- 5: -
- 6: U-Profil-Träger
- 7: Ende (6)
- 8: Ende (6)
- 9: Mittelteil
- 10: -
- 11: Mantel
- 12: Scheibe (2)
- 13: Scheibe (2)
- 14: Oberseite (6)
- 15: -
- 16: Druckprodukt
- 17: Meßtisch

- T: Zeit
- r: Radius (12, 13)
- R: Radius (11)

## Patentansprüche

1. Verfahren zur Beurteilung des Abschmierverhaltens von auf Papier gedruckter Farbe unter Verwendung einer in Kontakt zu einer bedruckten Fläche bringbaren abrollbaren Gegenfläche (11), dadurch gekennzeichnet, daß nach einer Festgesetzten Zeit (T) nach einem zuvor erfolgten Probedruck ein auf den Radius (r) einer schwer deformierbaren, farbabstoßenden kreiszylindrischen Scheibe (12; 13) zusammendrückbarer, deformierbarer, farbfreundlicher, kreiszylindrischer Mantel (11) größeren Radius (R) zum Abnehmen der Farbanteile auf der bedruckten Fläche ab- und zum Übertragen der abgenommenen Farbanteile zu einer unbedruckten Fläche weitergerollt wird, daß ein auf einer vorher unbedruckten Fläche erfolgter Abdruck visuell oder densitometrisch ausgewertet wird.

2. Vorrichtung zur Durchführung des Verfahrens nach Anspruch 1 in Anordnung nach einem Druckwerk einer Druckmaschine, dadurch gekennzeichnet, daß in einem mit einem Handgriff (3) versehenen U-Profil-Träger (6) eine Rolle (2) drehbar gelagert ist, und daß die Rolle (2) zwischen zwei aus einem farbabstoßenden Material bestehenden, schwer deformierbaren Scheiben (12, 13) einen farbfreundlichen, leicht deformierbaren Mantel (11) aufweist, wobei ein Radius (r) der Scheiben (12; 13) kleiner ist als ein Radius (R) des Mantels (11).

## Claims

1. Process for assessing the blotting behaviour of ink printed on paper using an unwindable opposing face (11) which can be brought into contact with a printed surface, characterised in that, a predetermined period of time (T) after previous experimental printing, a deformable, ink-friendly, circular cylindrical covering (11), which has a relatively great radius (R) and can be compressed to the radius (r) of an ink-repelling, circular cylindrical disc (12; 13) deformable with difficulty, is unwound in order to remove the ink components on the printed surface and is wound on in order to transfer the removed ink components to an unprinted surface, in that an impression made on a previously unprinted surface is evaluated visually or by density measurement.

2. Apparatus for carrying out the process according to claim 1 arranged after a printing unit of a printing machine, characterised in that a roller (2) is rotatably mounted in a U-profile carrier (6) provided with a handle (3) and in that the roller (2) has an ink-friendly, easily deformable covering (11) between two discs (12, 13) deformable with difficulty and consisting of an ink-repelling material, wherein a radius (r) of the discs (12, 13) is smaller than a radius (R) of the covering (11).

## Revendications

1. Procédé d'appréciation du comportement antimaculateur d'encre imprimée sur du papier, avec utilisation d'une contre-surface (11) pouvant rouler, susceptible d'être mise au contact d'une face imprimée, caractérisé en ce qu'à l'achèvement d'une durée (T) fixée, après une impression d'essai ayant été effectuée au préalable, on continue à faire rouler, sur le rayon (r) d'un disque (12; 13) de forme cylindrique circulaire, repoussant l'encre, difficilement déformable, une enveloppe (11) cylindrique circulaire, réceptive pour les encres et de plus grand rayon (R), pour enlever les quantités d'encre se trouvant sur la surface imprimée, les extraire, et pour transférer les parties d'encre enlevées à une surface non-imprimée, de manière qu'une impression, s'étant effectuée sur une surface qui n'était pas imprimée au préalable, fasse l'objet d'une évaluation visuelle ou densitométrique.

2. Dispositif pour mettre en oeuvre le procédé selon la revendication 1, monté en aval d'un groupe d'impression d'une machine à imprimer, caractérisé en ce qu'un rouleau (2) est monté de façon à pouvoir tourner dans un support à profil en grand U (6), pourvu d'une poignée (3), et en ce que le rouleau (2) présente entre deux disques (12, 13) difficilement déformables, constitués d'un matériau repoussant l'encre, une enveloppe (11) légèrement déformable, réceptive pour les encres, le rayon (r) du disque (12; 13) étant inférieur au rayon (R) de l'enveloppe (11).
